# EUROPEAN PATENT SPECIFICATION

(11) **EP 3 861 922 B1**
(45) Date of publication and mention of the grant of the patent: **18.10.2023**
(21) Application number: 18938666.7
(22) Date of filing: 21.12.2018
(51) Int. Cl.: A61B 1/317

(54) **ENDOSCOPE FOR USE IN MULTI-CHANNEL MINIMALLY INVASIVE CHANNEL**
ENDOSKOP ZUR VERWENDUNG IN EINEM MEHRKANALIGEN MINIMALINVASIVEN KANAL
ENDOSCOPE DESTINÉ À ÊTRE UTILISÉ DANS UN CANAL À INVASION MINIMALE MULTICANAL

(30) Priority: 01.11.2018 CN 201821789249 U
(43) Date of publication of application: 11.08.2021
(73) Proprietor: Dragon Crown Medical Co., Ltd., Jinan, Shandong 250101 (CN)
(72) Inventor: YANG, Wenzhou, Jinan, Shandong 250101 (CN)
(74) Representative: Sun, Yiming
(86) International application number: PCT/CN2018/000428
(87) International publication number: WO 2020/087191

(56) References cited:
- CN-A- 108 309 395
- US-A1- 2015 230 697
- US-A1- 2017 332 886

## Description

### TECHNICAL FIELD

The present invention relates to the field of surgical instrument and equipment, in particular to an endoscope for multi-channel minimally invasive channel.

Specifically, the present invention relates to an endoscope as defined by independent claim 1. Preferred embodiments are defined in the dependent claims.

### BACKGROUND

Document CN 108 309 395 A discloses an endoscope of the generic type as specified by the preamble of claim 1.

Document US 2015/0230697 A1 discloses a master-slave robotic endoscopy system including a flexible primary endoscope probe having at least one tool channel for carrying a tendon-sheath driven robot arm and corresponding end effector, and a secondary endoscope probe channel for carrying an imaging endoscope.

Document CN 108 309 395 A discloses a device for foramen intervertebral endoscope surgery. A working channel within the device comprises a main working channel and an auxiliary working channel which are connected in parallel; a foramen intervertebral endoscope is installed inside the main working channel, and a foramen intervertebral molding instrument is installed in the auxiliary working channel.

Minimally invasive surgery refers to a surgery performed by use of modern medical instruments such as laparoscope or thoracoscope, as well as a minimally invasive channel device and related equipment. Since the minimally invasive surgery has the advantages of less trauma, light pain, and fast recovery, the concept of "minimally invasive" has penetrated into various fields of surgical operations.

In minimally invasive techniques, a transforaminal endoscope may be used. The transforaminal endoscope includes an external sleeve and an endoscope provided inside the sleeve. The traditional endoscope is provided with a cleaning channel, an optical channel, an instrument channel, and an optical fiber therein. The optical channel is used to place a camera therein, such that an operator can observe the circumstance of an operation site. A surgical instrument is placed inside the instrument channel. However, the traditional scheme has the following shortcomings.
1. The surgical instruments are different in size, wherein the small-sized instrument is insufficient in mechanical strength, and the large-sized instrument cannot be inserted into the instrument channel smoothly and can only be used individually.
2. The optical channel is fixed on one side of the instrument channel, such that there is a blind spot in the case of observing the operation of the instrument.

### SUMMARY

The objective of the present invention is to provide an endoscope for a multi-channel minimally invasive channel, which can assist in observation when the endoscope is working.

In order to fulfill the above objective, the present invention provides the technical solution as defined in claim 1. Preferred embodiments of the invention are defined in dependent claims.

The accessory endoscope which is provided in the accessory channel has an observation function, that is, which can replace an observation function of the main channel and can also play an auxiliary role.

The present invention has the following beneficial effects:
1. Different transforaminal endoscopes can be placed in the main channel and the accessory channel respectively, and the operation site can be observed from multiple angles during the surgical operation.
2. Large-sized instruments can be placed in the main channel, such that instruments with higher strength can be used with a wider range of adaptation.
3. The accessory endoscope is specially used for observation during operation, and is not equipped with surgical instruments, so the volume is smaller, and space is saved.

### BRIEF DESCRIPTION OF THE DRAWINGS

In order to explain the technical solution of the present invention more clearly, the accompanying drawings that need to be used in the description will be briefly introduced below. Obviously, the accompanying drawings in the following description are merely some embodiments of the present invention. Those of ordinary skill in the art can obtain other accompanying drawings based on these accompanying drawings without paying a creative work.
FIG. 1 is a structural schematic diagram of the present invention;
FIG. 2 is a cross-sectional schematic diagram of a main endoscope;
FIG. 3 is a cross-sectional schematic diagram of an accessory endoscope;
FIG. 4 is a sectional view of a working channel in a direction A;
FIG. 5 is an enlarged drawing of a position B in Embodiment 1;
FIG. 6 is an enlarged drawing of a position B in Embodiment 2; and
FIG. 7 is a structural schematic diagram of Embodiment 3.

### Reference numerals:

1: working channel; 2: accessory endoscope; 3: main endoscope; 4: surgerical instrument; 11: main channel; 12: accessory channel; 13: handle; 14: main channel outlet; 15: endoscope view hole; 16: accessory channel outlet; 17: reinforcing rib; 21: accessory cleaning channel; 22: accessory optical channel; 31: main cleaning channel; 32: main optical channel; 33: main instrument channel.

### DETAILED DESCRIPTION

In order for those skilled in the art to better understand the technical solution of the present invention, the present invention will be further described in detail below in conjunction with the accompanying drawings.

### Embodiment one

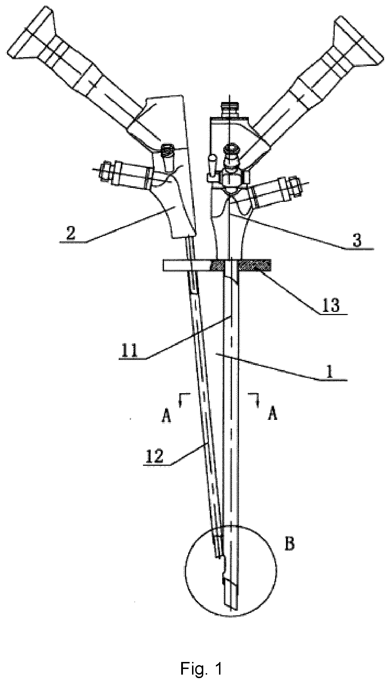

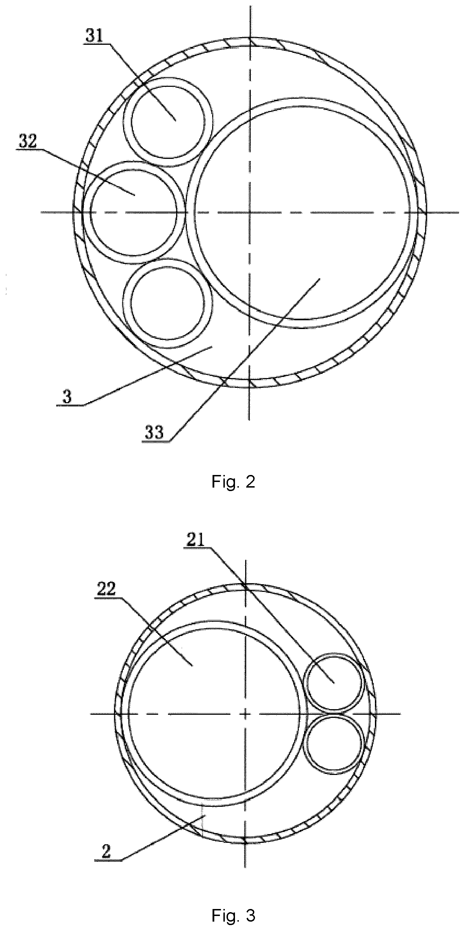

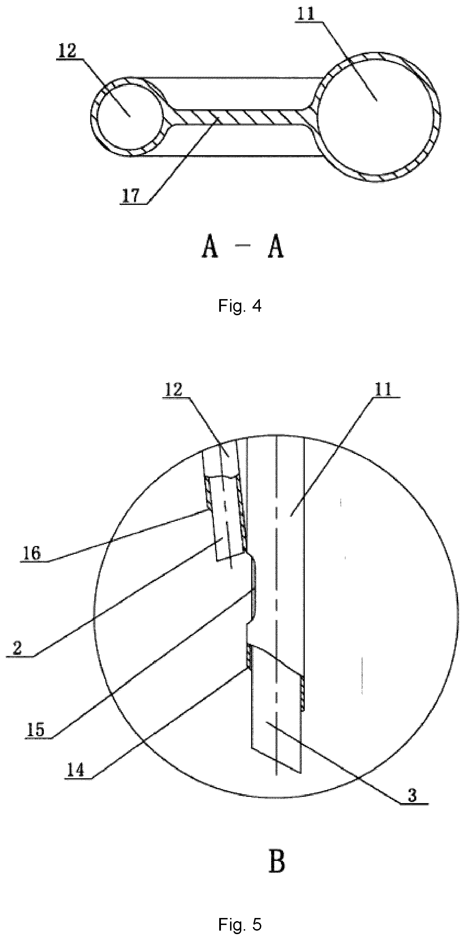

As shown in FIGS. 1-5, this embodiment of the present invention provides an endoscope for a multi-channel minimally invasive channel. The endoscope comprises a working channel 1, an accessory endoscope 2 and a main endoscope 3. The working channel 1 comprises a main channel 11, an accessory channel 12 and a handle 13 which are integrally provided, wherein the main channel 11 and the accessory channel 12 are connected via the handle 13, and inlets of the main channel 11 and the accessory channel 12 are both provided at the handle 13. One end of the main channel 11 away from the handle 13 is provided with a main channel outlet 14 and an endoscope view hole 15, and the accessory channel 12 is also provided with an accessory channel outlet 16. The endoscope view hole 15 is provided at one side of the main channel outlet 14, and is located between the main channel outlet 14 and the accessory channel outlet 16. The main channel 11 and the accessory channel 12 are arranged at an acute angle, and are separated at their inlets and tightly attached at their outlets, that is, the accessory channel outlet 16 is closely attached to the main channel 11. The length of the main channel 11 is greater than that of the accessory channel 12.

The main channel 11 and the accessory channel 12 are also connected via a reinforcing rib 17.

The main endoscope 3 is of a traditional endoscope structure, and is provided with a main cleaning channel 31, a main optical channel 32, and a main instrument channel 33 therein. The accessory endoscope 2 is provided with an accessory cleaning channel 21 and an accessory optical channel 22 therein, and no instrument channel is provided inside the accessory endoscope 2. The main endoscope 3 is provided in the main channel 11, and the accessory endoscope 2 is provided in the accessory channel 12.

The working principle of the present invention is as follows:
during working, the main endoscope 3 and the accessory endoscope 2 are placed in the main channel 11 and the accessory channel 12 respectively, and this endoscope device is then used for a surgical operation. Both the main endoscope 3 and the accessory endoscope 2 have optical channels and can be switched for observation.

### Embodiment two

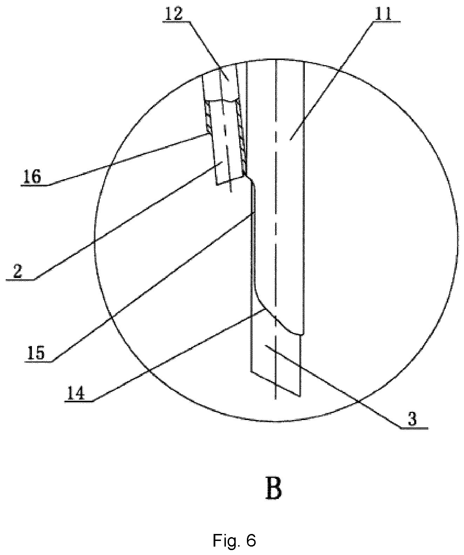

As shown in FIG. 6, in this embodiment, the endoscope view hole 15 and the main channel outlet 14 are integrally provided, which can further increase range of motion of an instrument and facilitate observation of the instrument by the accessory endoscope 2. The other parts of this embodiment are basically same as Embodiment one.

### Embodiment three

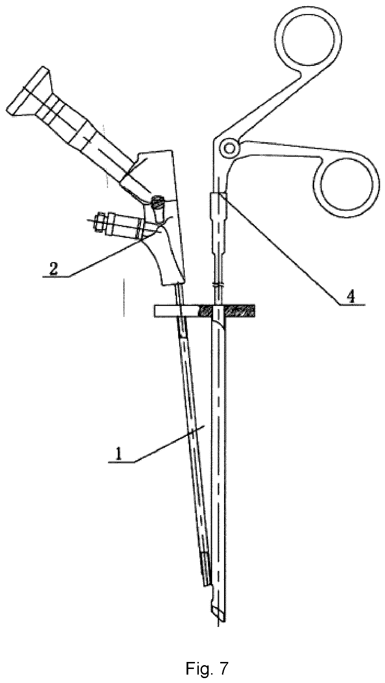

As shown in FIG. 7, in this embodiment, a surgical instrument 4, rather than the main endoscope 3, is directly placed in the main channel 11. Therefore, a larger-sized instrument can be directly used, which increases the versatility in case of the channel diameter being inconvenient. The accessory endoscope 2 can also be used for cleaning and image observation. The other parts of this embodiment are basically same as Embodiment one.

The above content has only described some exemplary embodiments of the present invention by way of illustration. Therefore, the above accompanying drawings and description are illustrative in nature and should not be construed as limiting the protection scope of the claims.

## Claims

1. An endoscope for a multi-channel minimally invasive channel, comprising: a working channel (1) , and an accessory endoscope (2), wherein the working channel (1) comprises a main channel (11), an accessory channel (12) and a handle (13), and the main channel (11), the accessory channel (12) and the handle (13) are rigidly connected; wherein inlets of the main channel (11) and the accessory channel (12) are provided on the handle (13); wherein the accessory endoscope (2) is provided in the accessory channel (12);
wherein a length of the main channel (11) is greater than that of the accessory channel (12); wherein the main channel (11) and the accessory channel (12) are arranged within the working channel (1) at an acute angle, and the main channel (11) and the accessory channel (12) are attached at their outlets and separated at their inlets; and the main channel (11) and the accessory channel (12) are also connected via a reinforcing rib (17), **characterized in that** the endoscope comprises a main endoscope (3) provided in the main channel (11), and a main cleaning channel (31), a main optical channel (32) and a main instrument channel (33) are provided in the main endoscope (3); and
the accessory endoscope (2) is provided with an accessory cleaning channel (21) and an accessory optical channel (22) therein; and no instrument channel is provided in the accessory endoscope (2).

2. The endoscope according to claim 1, **characterized in that** an endoscope view hole (15) is provided at one side of the main channel outlet (14), and located between the main channel outlet (14) and the accessory channel outlet (16).

3. The endoscope according to claim 2, **characterized in that** the main channel (11), the accessory channel (12) and the handle (13) are of an integrated structure.

4. The endoscope according to claim 1, **characterized in that** the main channel outlet (14) communicates with the endoscope view hole (15).

5. The endoscope according to claim 1, **characterized in that** the diameter of the main channel (11) is greater than that of the accessory channel (12).

## Patentansprüche

1. Endoskop für einen mehrkanaligen minimalinvasiven Kanal, das umfasst: einen Arbeitskanal (1), und ein Begleitendoskop (2), wobei der Arbeitskanal (1) einen Hauptkanal (11), einen Begleitkanal (12) und einen Griff (13) umfasst, und wobei der Hauptkanal (11), der Begleitkanal (12) und der Griff (13) starr miteinander verbunden sind; wobei Eingänge des Hauptkanals (11) und des Begleitkanals (12) auf dem Griff (13) bereitgestellt sind; wobei das Begleitendoskop (2) in dem Begleitkanal (12) bereitgestellt ist;
wobei eine Länge des Hauptkanals (11) größer als die des Begleitkanals (12) ist; wobei der Hauptkanal (11) und der Begleitkanal (12) innerhalb des Arbeitskanals (1) unter einem spitzen Winkel angeordnet sind, und wobei der Hauptkanal (11) und der Begleitkanal (12) an ihren Ausgängen befestigt und an ihren Eingängen getrennt sind; und wobei der Hauptkanal (11) und der Begleitkanal (12) auch über eine Verstärkungsrippe (17) verbunden sind, **dadurch gekennzeichnet, dass**
das Endoskop ein Hauptendoskop (3) umfasst, das in dem Hauptkanal (11) bereitgestellt ist, und dass ein Hauptreinigungskanal (31), ein Haupt-Optikkanal (32) und ein Haupt-Instrumentenkanal (33) in dem Hauptendoskop (3) bereitgestellt sind; und dass
das Begleitendoskop (2) mit einem Begleitreinigungskanal (21) und mit einem Begleit-Optikkanal (22) darin versehen ist; und dass kein Instrumentenkanal in dem Begleitendoskop (2) bereitgestellt ist.

2. Endoskop nach Anspruch 1, **dadurch gekennzeichnet, dass** ein Endoskopsichtloch (15) an einer Seite des Hauptkanalausgangs (14) bereitgestellt und zwischen dem Hauptkanalausgang (14) und dem Begleitkanalausgang (16) angeordnet ist.

3. Endoskop nach Anspruch 2, **dadurch gekennzeichnet, dass** der Hauptkanal (11), der Begleitkanal (12) und der Griff (13) aus einer einstückigen Struktur bestehen.

4. Endoskop nach Anspruch 1, **dadurch gekennzeichnet, dass** der Hauptkanalausgangs (14) mit dem Endoskopsichtloch (15) in Verbindung steht.

5. Endoskop nach Anspruch 1, **dadurch gekennzeichnet, dass** der Durchmesser des Hauptkanals (11) größer als der des Begleitkanals (12) ist.

## Revendications

1. Endoscope pour un canal à invasion minimale à multi-canaux, comprenant : un canal de travail (1) et un endoscope accessoire (2), dans lequel le canal de travail (1) comprend un canal principal (11), un canal accessoire (12) et une poignée (13), et le canal principal (11), le canal accessoire (12) et la poignée (13) sont connectés de manière rigide ; dans lequel des entrées du canal principal (11) et du canal accessoire (12) sont prévues sur la poignée (13) ; dans lequel l'endoscope accessoire (2) est prévu dans le canal accessoire (12) ;
dans lequel une longueur du canal principal (11) est supérieure à celle du canal accessoire (12) ; dans lequel le canal principal (11) et le canal accessoire (12) sont agencés à l'intérieur du canal de travail (1) à un angle aigu, et le canal principal (11) et le canal accessoire (12) sont fixés à leurs sorties et séparés à leurs entrées ; et le canal principal (11) et le canal accessoire (12) sont également raccordés par le biais d'une nervure de renfort (17), **caractérisé en ce que**
l'endoscope comprend un endoscope principal (3) prévu dans le canal principal (11), et un canal de nettoyage principal (31), un canal optique principal (32) et un canal d'instrument principal (33) sont prévus dans l'endoscope principal (3) ; et
l'endoscope accessoire (2) est doté d'un canal de nettoyage accessoire (21) et d'un canal optique accessoire (22) à l'intérieur ; et aucun canal d'instrument n'est prévu dans l'endoscope accessoire (2).

2. Endoscope selon la revendication 1, **caractérisé en ce qu'**un trou de vue d'endoscope (15) est prévu sur un côté de la sortie de canal principal (14), et situé entre la sortie de canal principal (14) et la sortie de canal accessoire (16).

3. Endoscope selon la revendication 2, **caractérisé en ce que** le canal principal (11), le canal accessoire (12) et la poignée (13) sont d'une structure intégrée.

4. Endoscope selon la revendication 1, **caractérisé en ce que** la sortie de canal principal (14) communique avec le trou de vue d'endoscope (15).

5. Endoscope selon la revendication 1, **caractérisé en ce que** le diamètre du canal principal (11) est supérieur à celui du canal accessoire (12).
